# EUROPEAN PATENT APPLICATION

(11) **EP 1 560 023 A1**
(43) Date of publication of application: **03.08.2005**
(21) Application number: 03714619.8
(22) Date of filing: 24.03.2003
(51) Int. Cl.: G01N 33/53, G01N 33/543, G01N 33/68

(54) **A PROTEIN CHIPS DETECTING SYSTEM WHICH CAN SIMULTANEOUSLY DETECT MULTI TARGET**

(30) Priority: 24.10.2002 CN 02137620
(71) Applicant: Shanghai Health Digit Co. Limited, 200233 Shanghai (CN)
(72) Inventor: HU, Gengxi, Shanghai 200233 (CN)
(74) Representative: Bublak, Wolfgang
(86) International application number: PCT/CN2003/000208
(87) International publication number: WO 2004/038413

(57) **Abstract**

The present invention provides a protein chips detecting system which can simultaneously detect multi target, and the method for manufacturing the system. The invention increases the stability and sensitivity of the system by improving the present protein chips system and the manufacture.

## Description

### Field of the Art

The present invention relates to biotechnology. More specifically, it relates to a protein chip detecting system for parallel detection of multiple indices and preparation of the same.

### Background of the Art

As a new biotechnology emerging in the middle of 1990s, biochip technology is based on large-scale parallel analysis of the interaction between biomacro-molecules (e.g., nuclear acids, proteins, etc.). It combines many other technologies, such as microelectronics, micromechanics, chemistry, physics, computer science and the like, to serialize, integrate and miniaturize the processes of sample reaction, detection, analysis and the like. It has become one of the technologies in life sciences that see the fastest development today.

Biochips can be widely applied in agriculture, environment, food, courts, military affairs, research and the like. Further, since biochips can be used to examine several disease concerning genes or proteins at the same time, so as to aid the diagnosis of genetic diseases, tumor, infectious diseases and the like, they have great value in terms of clinic application.

Biochips fall into three maior categories: nuclear acid chips, protein chips and chip labs.

A protein chip is a biochip for the detection of the interaction between proteins. The protein chip technology under current research is mainly based on the principle of specific binding of antigen and antibody. According to the protein chip technology, several proteins are bonded to the solid substrate (e.g., specially treated slides, organic films, silicon microspheres, etc.), and the corresponding proteins in the biological samples that can specifically combine said several proteins are detected.

By detecting the content change of particular intrinsic protein, a protein chip can accurately determine the physiological/pathological change. Protein chips have a significant potential for application in clinic diagnosis.

In Chinese patent application CN 01105023.3, the present inventors have disclosed a detecting system for simultaneous detection of multiple indices of a protein chips and the preparation of the same, so that the proteins which are highly specific but have low content in body fluid may be detected simultaneously in clinic.

### Disclosure of the Invention

The technical problem to be solved by the present invention is, based on the original application CN 01105023.3, to improve the protein chip detecting system, so as to increase its stability and sensitivity.

Chinese patent application CN 01105023.3 has disclosed a protein chip detecting system for parallel detection of multiple indices, which includes:
(1) a protein chip for parallel detection of multiple indices;
(2) a mixed solution of two or more proteins, i.e. reactive solution, prepared in a particular ratio of concentrations and bearing luminescent labels;
(3) a series of mixed solutions of proteins to be detected at known and increasing concentrations, i.e. standard samples;
(4) a solution for rinsing protein chips.

The present invention modified the formula of the standard sample solutions of the proteins to be detected, so as to prevent the deactivation of different proteins after mixing. As a result, both the stability of the sample and the accuracy of the detection are increased.

The formulae of said standard sample solutions of the proteins to be detected (or briefly called target proteins A) at known and increasing concentrations are as follows: 40%-60% fetal bovine serum + various highly concentrated purified antigens + 0.02-0.1‰ NaN₃, or pH 7.0-7.8 0.05M PB (KH₂PO₄-Na₂HPO₄) + 2-30% BSA + 1.5-2.5% sucrose + 0.02-0.1‰ NaN₃ + various highly concentrated purified antigens. The series of mixed solutions as prepared above that include target proteins A at known and increasing concentrations are lyophilized and stored.

Another technical problem to be solved by the present invention is to improve the method for preparing the protein chip in the above detecting system.

The protein chip as described in the present invention includes a solid phase support and proteins immobilized on the support for parallel detection of multiple indices. The protein chip utilizes automated chip spotting system to immobilize various proteins on the solid phase support in a particular array by way of physical adsorption or covalent bonding; then a blocking solution is used to block the positions that have no sample spotted on the solid phase support, and the resulting product is dried and stored. The proteins may be antigens, antibodies, receptors, ligands and the like. They further include proteins that can specifically bind to intrinsic disease-marker proteins, in particular tumor marker proteins.

Said solid phase support may be an inorganic substrate or an organic compound substrate. The inorganic substrates include semiconducting silicon substrates, glass substrates, microporous silicon substrates, microporous glass substrates and the like. Glass substrates are preferred. The organic substrates include cellulose acetate films, cellulose nitrate films, nylon films, polypropylene films and the like.

The protein chip is obtained by the following technical scheme:
1. Various proteins to be detected (briefly called target proteins A) and the antigens, antibodies or receptors and the like (briefly called B) which are specific for A, such as endogenous disease marker proteins and the proteins that can bind to them, are determined.
2. B as described above, i.e. various protein probes are dissolved in a coating solution in particular concentration, and then are spotted on the solid phase support by automated chip spotting system, wherein the spotting density is 25-200 spots/cm², and the spotting amount is 0.1-10ng/spot.
3. The resulting product is placed overnight at 4°C.
4. The protein chip is blocked with a blocking solution.
5. The protein chip is dried and stored at 4°C.

In order to locate on neighboring orders of magnitude (the strongest spot/the weakest spot < 100) the photo signals which are generated by various proteins spotted on the chip after a series of reactions, the concentrations of the proteins on the chips are adjusted in advance for the sake of convenient detection by the detecting instrument (to obtain the optimum spotting concentrations).

The formula of the coating solution provided by the present invention is as follows: PB (KH₂PO₄-Na₂HPO₄), pH 7.0-8.0.

The formula of the blocking solution provided by the present invention is as follows: PB(KH₂PO₄-Na₂HPO₄) containing 1-9% BSA, 1-9% sucrose and 0.01-1‰ NaN₃, the advantages of which include that BSA has the function of blocking, sucrose is inert enough to isolate air, and BSA as well as sucrose can support the framework. The role of blocking solution is to prevent the other positions on the solid phase support from combining the proteins, so that the accuracy of the experimental data can be ensured.

Compared with that in the prior invention, the modified formula of the buffer solution used in the method of preparation according to the present invention facilitates the decrease of the non-specific adsorption and the background signal values. It also increases the stability of the protein chip and the detecting sensitivity.

### Description of the Drawings

Fig. 1 illustrates the signal generated by the protein chip prepared with the method using the modified buffer solution formula according to the present invention.
Fig. 2 illustrates the signal generated by the protein chip prepared with the method according to the prior invention.

### Specific Embodiments

As used in the present invention, the amount of the solid materials (e.g., BSA, sucrose, tyrosine and NaN₃) is presented in weight percent, and that of the solutions (e.g. Tween 20, fetal bovine serum and Proclin solution) is in volume percent.

Example 1 Comparison of the background and signal values between the protein chips for detection of tumor prepared with the formula including the modified buffer solution (including blocking solution and coating solution) and the protein chips prepared with the formula including the prior buffer solution

### 1. Preparation of protein chip E with the formula including the modified buffer solution:

1.1 The corresponding antibodies (antibodies I) of six tumor markers, i.e., AFP, CEA, PSA, free-PSA, CA125, CA15-3, were dissolved in a coating solution at particular concentrations, and then these proteins were spotted on the solid phase support by using automated chip spotting system, wherein the spotting density was 48 spots/cm², the spotting amount was 0.5ng/spot, and each antibody in antibodies I corresponded to 4 parallel spots;
1.2 The resulting product was placed overnight at 4°C;
1.3 The protein chip was treated with a blocking solution for one hour;
1.4 The protein chip was dried for 2 hours and was stored at 4°C for future use.

The formula of the coating solution used above was PB (KH₂PO₄-Na₂HPO₄), pH 7.0-8.0.

The formula of the blocking solution used above was: PB (KH₂PO₄-Na₂HPO₄) containing 3% BSA, 5% sucrose and 0.5‰ NaN₃.

### 2. Preparation of protein chip F with the prior method for preparing protein chips

The formula of the coating solution used therein was CBS (NaHCO₃-Na₂CO₃), pH 9.6.

The formula of the blocking solution used therein was TBS containing 0.2% Tween20, 0.1% tyrosine, 5% BSA, 4% sucrose, 0.5% Proclin.

The protein chips E and F as described above reacted with the corresponding reaction solutions, rinsing solutions and serum samples at known concentrations (the remaining serum samples were left for use in next experiment). The resulting products were photographed with a biochip detecting instrument, and the data obtained were analyzed. The signal values were shown in Table 1:

**Table 1**

| | Chip E | Chip F |
|---|---|---|
| AFP | 925 | 1035 |
| CEA | 2995 | 2906 |
| PSA | 2170 | 2201 |
| Free-PSA | 2017 | 1978 |
| CA125 | 913 | 879 |
| CA153 | 1082 | 1033 |

After stored at 4°C for six months, the chips as described above reacted with the reaction solutions and the rinsing solutions from the same batch as that in the above experiment, as well as the remaining serum samples as described above, and the signal values were shown in Table 2:

**Table 2**

| | Chip E | Chip F |
|---|---|---|
| AFP | 875 | 477 |
| CEA | 2630 | 1486 |
| PSA | 2140 | 864 |
| Free-PSA | 1087 | 671 |
| CA125 | 906 | 311 |
| CA153 | 972 | 557 |

Compared with protein chip F, protein chip E prepared with the formula of the buffer solution according to the present invention has an obviously better stability.

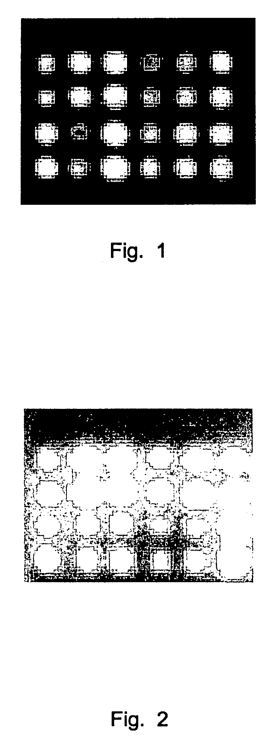

Further, the signal of the protein chip E derived from the biochip photograph is shown in Fig. 1, and that of protein chip F is shown in Fig. 2. It can be seen from these two figures that the background of Fig. 1 is much lower than that of Fig. 2.

Example 2 Comparison of the stability between the standard sample prepared with the modified formula of the buffer solution and the prior standard sample
1. The buffer solution for the standard sample was prepared in accordance with the following formula: 60% fetal bovine serum + various highly concentrated purified antigens + 0.05‰ NaN₃. Standard sample solution A was obtained with further addition of 60µl highly concentrated purified antigens AFP, CEA, NSE;
2. The buffer solution for the standard sample was prepared in accordance with the following formula: pH 7.8 0.05M PB (KH₂PO₄-Na₂HPO₄) + 15% BSA + 2.5% sucrose + 0.05‰ NaN₃. Standard sample solution B was obtained with further addition of 60µl highly concentrated purified antigens AFP, CEA, NSE;
3. The buffer solution for the standard sample was prepared in accordance with the prior formula, and standard sample solution C was obtained with further addition of 60µl highly concentrated purified antigens AFP, CEA, NSE.

Note: The final concentrations of AFP, CEA and NSE in solutions A, B and C were consistent.

The above three solutions of standard samples were lyophilized (standard samples a, b and c) and were determined with auto-analyzer of chemluminescence. The concentrations as determined were given in Table 3:

**Table 3**

| Tumor marker | unit | Standard sample a | Standard sample b | Standard sample c |
|---|---|---|---|---|
| AFP | ng/ml | 728 | 709 | 711 |
| NSE | ng/ml | 1862 | 1830 | 1875 |
| CEA | ng/ml | 1161 | 1163 | 1172 |

After stored at 4 °C for three months, standard samples a, b and c were re-determined with the same auto-analyzer of chemluminescence, and the concentrations as determined were shown in Table 4:

**Table 4**

| Tumor marker | unit | Standard sample a | Standard sample b | Standard sample c |
|---|---|---|---|---|
| AFP | ng/ml | 731 | 705 | 352 |
| NSE | ng/ml | 1799 | 1803 | 1007 |
| CEA | ng/ml | 1095 | 1168 | 409 |

Compared with that of standard sample c prepared with the prior buffer system, the concentrations of standard sample a and b are more stable, so that they can be stored for a longer time without losing activity.

## Claims

1. A protein chip detecting system for parallel detection of multiple indices, comprising
a protein chip for parallel detection of multiple indices,
a mixed solution of two or more proteins prepared in a particular ratio of concentrations and bearing luminescent labels, i.e., reactive solution,
a series of mixed solutions of proteins to be detected at known and increasing concentrations, i.e. standard samples solutions, and
a solution for rinsing protein chips,
wherein the formulae of said standard sample solutions are as follows: 40%-60% fetal bovine serum, various highly concentrated purified antigens, and 0.02-0.1‰ NaN₃, or
pH 7.0-7.8 0.05M PB (KH₂PO₄-Na₂HPO₄), 2-30% BSA, 1.5-2.5% sucrose, 0.02-0.1‰ NaN₃ and various highly concentrated purified antigens.

2. A method for preparing the protein chip detecting system as described in claim 1, wherein the protein chip is obtained by the following steps:
1) various proteins to be detected (briefly called target protein A) and antigens, antibodies or receptors and the like specific for A (briefly called B), such as endogenous disease-marker proteins and proteins that can specifically bind to them, are determined;
2) B as described above, i.e. various protein probes are dissolved in a coating solution in particular concentration, and then are spotted onto the solid phase support by automated chip spotting system, wherein the spotting density is 25-200 spots/cm² and the spotting amount is 0.1-10ng/spot;
3) the resulting product is placed overnight at 4°C;
4) the protein chip is blocked with a blocking solution;
5) the protein chip is dried and stored at 4°C,
wherein the formula of said coating solution is as follows: PB (KH₂PO₄-Na₂HPO₄), pH 7.0-8.0.

3. A method for preparing the protein chip detecting system as described in claim 2, wherein the formula of said blocking solution is PB (KH₂PO₄-Na₂HPO₄) containing 1-9% BSA, 1-9% sucrose and 0.01-1‰ NaN₃.
